(19) Europäisches Patentamt
European Patent Office
Office européen des brevets

(11) Veröffentlichungsnummer: **0 015 437**
**B1**

(12) # EUROPÄISCHE PATENTSCHRIFT

(45) Veröffentlichungstag der Patentschrift: **25.11.81**

(51) Int. Cl.³: **C 12 Q 1/50,** C 12 Q 1/66

(21) Anmeldenummer: **80100825.1**

(22) Anmeldetag: **19.02.80**

(54) **Verfahren und Reagenz zur Bestimmung der Creatinkinase.**

(30) Priorität: **02.03.79 DE 2908054**

(43) Veröffentlichungstag der Anmeldung: **17.09.80 Patentblatt 80/19**

(45) Bekanntmachung des Hinweises auf die Patenterteilung: **25.11.81 Patentblatt 81/47**

(84) Benannte Vertragsstaaten: **AT BE CH DE FR GB IT LU NL SE**

(56) Entgegenhaltungen:
**FR-A-2 214 887**
**GB-A-2 026 156**
**US-A-3 423 290**
**US-A-4 001 088**
**BIOCHEMICAL JOURNAL, Band 57, 1954, A. H. ENNOR et al.: »Some properties of creatine phosphokinase«, Seiten 203–212**
**CHEMICAL ABSTRACTS Band 91, Nr. 11, 10. September 1979, Seite 311, Zusammenfassung Nr. 86090t, Columbus, Ohio, US, A. LUNDIN: »Determination of creatine kinase isoenzymes in human serum by an immunological method using purified firefly luciferase«**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Wulff, Karl, Dr., Pütrichstrasse 18, D-8120 Weilheim (DE)**
Erfinder: **Stähler, Fritz, Dr. med., Heimgartenstrasse 4, D-8132 Tutzing (DE)**
Erfinder: **Gruber, Wolfgang, Dr., Am Oberanger 7, D-8132 Tutzing-Unterzeismering (DE)**

(74) Vertreter: **Weickmann, Heinrich, Dipl.-Ing Patentanwälte Dipl.Ing.H.Weickmann et al, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)**

**EP 0 015 437 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren und Reagenz zur Bestimmung der Creatinkinase

Die Erfindung betrifft ein Verfahren und ein Reagenz zur photometrischen Aktivitätsbestimmung von Creatinphosphokinase (e.C. 2.7.3.2), im folgenden CK genannt, mit Hilfe des Biolumineszenz-Systems aus Leuchtkäfern.

Die CK katalysiert die folgende Reaktion:

$$\text{ADP} + \text{Creatinphosphat} \underset{}{\overset{\text{CK}}{\rightleftharpoons}} \text{ATP} + \text{Creatin} \qquad (1)$$

Im menschlichen Körper kommen zwei verschiedene Arten von Untereinheiten dieses Enzyms vor, die Untereinheiten M und B. Da das aktive Enzym aus jeweils zwei Untereinheiten zusammengesetzt ist, und da die beiden Untereinheiten frei miteinander kombinieren können, sind drei Enzymtypen möglich, der Muskeltyp (CK—MM), der Gehirntyp (CK—BB) und der Hybridtyp (CK—MB), der praktisch nur im Myokard vorkommt, beim Herzinfarkt ins Serum austritt und dort in erhöhter Konzentration zu messen ist. Die Aktivität dieses Isoenzyms ist neben der Gesamtaktivität der CK im Serum für die Diagnose des Herzinfarkts von großer Bedeutung.

Die CK-Aktivität wird üblicherweise nach einem absorptionsphotometrischen Verfahren über die Messung der ATP-Bildung (vgl. Gleichung 1) wie folgt bestimmt:

$$\text{ATP} + \text{D-Glucose} \overset{\text{Hexokinase}}{\rightleftharpoons} \text{ADP} + \text{D-Glucose-6-phosphat} \qquad (2)$$

$$\text{D-Glucose-6-phosphat} + \text{NADP}^+ \xrightarrow{\text{G-6-PDH}} \text{D-Gluconat-6-phosphat} + \text{NADPH} + \text{H}^+ \qquad (3)$$

Bei dieser Bestimmung der CK-Aktivität über das gebildete ATP stören die Adenylatkinasen (E.C. 2.7.4.3 »Myokinasen«) des Serums (aus Leber, Muskel oder Erythrozyten stammend), die die folgende Reaktion katalysieren:

$$2\ \text{ADP} \overset{\text{Myokinase}}{\rightleftharpoons} \text{ATP} + \text{AMP} \qquad (4)$$

Ihre Aktivität läßt sich am wirksamsten durch Zugabe von 5 bis 10 mMol/l Adenosin-5'-monophosphat (AMP) und 10 µMol/l Diadenosinpentaphosphat (Ap5A) hemmen, wodurch die Störung des absorptions-photometrischen Tests beseitigt wird. Eine Beseitigung der Störung ist auch möglich, indem 1 bis 10 mMol/l NaF anstelle von Ap5A eingesetzt werden.

Ein wesentlicher Nachteil dieser Methoden ist eine lag-Phase, die sich nicht unter 90 Sekunden verringern läßt. Ein weiterer wesentlicher Nachteil der absorptions-photometrischen Methode liegt in ihrer geringen Empfindlichkeit, die nur eine Messung von Aktivitäten über 10 U/l erlaubt, und daher insbesondere eine Messung der CK—MB-Aktivität im unteren pathologischen Bereich sowie im Normalbereich praktisch unmöglich macht.

Es sind daher verschiedentlich Versuche unternommen worden, die ATP-Bildung der CK über die Firefly-Luciferase-Biolumineszenz zu verfolgen. Diese Methode hat den Vorteil größerer Empfindlichkeit und fehlender lag-Phase.

Die Luciferase aus der Firefly (Photinus pyralis u. a.) katalysiert die folgende Reaktion:

$$\text{ATP} + \text{Luciferin} + O_2 \xrightarrow{\text{Luciferase}} \text{Oxyluciferin} + CO_2 + \text{AMP} + PP_i + \text{Licht} \qquad (5)$$

Das bei dieser Reaktion entstehende Licht wird mit einer Ausbeute von nahezu 1 Einstein/Mol ATP emittiert. Es hat eine Wellenlänge im Maximum von 562 nm. Die Reaktion ist extrem empfindlich und erlaubt die quantitative Bestimmung von ATP-Konzentrationen bis herab zu $10^{-13}$ Mol/l.

Es ist jedoch bekannt, daß die Firefly-Reaktion 5 durch AMP so stark inhibiert wird (Arch. Biochem. Biophys. 141, 49 1970), daß eine praktische Anwendung dieser Reaktion für die Messung von CK-Aktivitäten gemäß Gleichung 1 im Serum sehr problematisch ist, da sie die Verwendung von AMP als Inhibitor der Adenylatkinase in Reaktion 4 verhindert (Proc. Nat. Acad. Sci. 71, 1384 bis 1387 {1974}). Es ist weiter bekannt, die Notwendigkeit der Hemmung der Adenylatkinase dadurch teilweise zu vermeiden, daß man die Reaktion 1 bei suboptimaler ADP-Konzentration, das heißt, ohne Substratsättigung, durchführt und so bei einer Substratkonzentration arbeitet, die der aufgrund der steileren Aktivitäts-Substraktkonzentrations-Kurve der Adenylatkinase die Aktivität der Adenylatkinase gegenüber der der CK vernachlässigbar klein ist. Auf diese Weise läßt sich zwar die CK im Serum noch neben Adenylatkinase mit der Biolumineszenzmethode messen (Clin. Chim. Acta 87, 199 bis 209 {1978}), jedoch muß man bekanntlich dabei erhebliche Nachteile in Kauf nehmen·

1. Die CK-Reaktion läuft nicht mehr nach pseudonullter Ordnung ab.
2. Die wiedergefundene CK-Aktivität, bezogen auf eine interne Eichung mit ATP, beträgt nur etwa 11% der im absorptions-photometrischen Test gemessenen Werte.
3. Geringfügige Ungenauigkeiten in der ADP-Konzentration gehen sehr stark in das Ergebnis ein.

Der vorliegenden Erfindung liegt daher die Aufgabe zugrunde, ein Biolumineszenz-Testverfahren zur Bestimmung der CK-Aktivität zu schaffen, das diese Nachteile nicht aufweist und bei dem insbesondere

1. die Adenylatkinasen voll hemmbar sind,
2. die über Biolumineszenz gemessene Aktivität, ausgedrückt in I.U. (μMol Substratumsatz/Minute), gleich der im absorptions-photometrischen Ansatz gemessenen Aktivität ist und
3. unter Bedingungen der Substratsättigung der CK gearbeitet werden kann.

Das erfindungsgemäße Verfahren zur Bestimmung von Creatinkinase durch Reaktion von Creatinphosphat mit Adenosindiphosphat unter Bildung von Adenosintriphosphat, Umsetzung des letzteren mit Luciferin und Sauerstoff in Gegenwart von Luciferase und Diadenosinpentaphosphat unter Bildung von Oxyluciferin und Adenosinmonophosphat, und Messung des dabei emittierten Lichts ist dadurch gekennzeichnet, daß man die Umsetzung bei Adenosindiphosphoat-Substrat-Sättigungskonzentration in Gegenwart von 1 bis 10 mMol/l AMP bei pH-Werten von 5,8 bis 7,5 mit mindestens 50 U/Test Luciferase durchführt.

Überraschenderweise werden unter den oben angegebenen Bedingungen nicht nur die Adenylatkinasen vollständig gehemmt, sondern die Luciferasereaktion läuft nach pseudoerster Ordnung ab, ohne daß sich eine Hemmung durch AMP nachteilig bemerkbar macht.

Vorzugsweise wird das Verfahren der Erfindung bei einem pH-Wert zwischen 6,3 und 7,2 durchgeführt, besonders bevorzugt zwischen pH 6,5 und 6,9. Die ADP-Konzentration je Test beträgt vorzugsweise 0,1 bis 10 mMol/l, die Konzentration an Diadenosinpentaphosphat (Ap5A) beträgt vorzugsweise 1 bis 100 μMol/l.

Im oben angegebenen bevorzugten pH-Wertbereich erhält man die besten Ergebnisse in Gegenwart von 0,5 bis 2 mMol/l ADP, 5 bis 50 μMol/l Ap5A und 5 bis 10 mMol/l AMP.

Außer den oben schon angegebenen Reagenzien wird beim erfindungsgemäßen Verfahren vorzugsweise auch eine organische Sulfhydrylverbindung zugesetzt. Geeignete Sulfhydrylverbindungen sind beispielsweise N-Acetylcystein, Dithiothreit, Dithioerythrit und reduziertes Glutathion. Bevorzugt wird N-Acetylcystein verwendet. Des weiteren wird zweckmäßig ein Sequestrierungsmittel zugesetzt, wie Äthylendiamin-tetraessigsäure (EDTA), Trilone, Komplexone, Sequestrene und dergleichen. Auch Stabilisierungsmittel, wie Serumalbumin, werden zweckmäßigerweise in dem Fachmann für die Stabilisierung enzymatischer Reagenzien bekannten Mengen zugesetzt.

Bei der Firefly-Reaktion ist die Intensität des emittierten Lichts der ATP-Konzentration direkt proportional. Die Meßgröße hat die Dimension einer Reaktionsgeschwindigkeit. Für eine Reaktion pseudoerster Ordnung ($C_{ATP} \ll K_m$) gilt:

$$I = \frac{d(h \cdot v)}{dt} = \frac{V_{max}}{K_m} \cdot C_{ATP}$$

Wird dieser Reaktion eine ATP-liefernde Reaktion, wie z. B. die CK-Reaktion vorgeschaltet, so gilt

$$\frac{d\,C_{ATP}}{dt} \sim \frac{dI}{dt}$$

d. h. es resultiert ein linearer Anstieg der Lichtintensität, dessen Steigung der Aktivität der eingesetzten CK proportional sein sollte.

Diese enzymkinetischen Überlegungen setzen voraus, daß sich die Aktivität der Luciferase innerhalb des Beobachtungszeitraums nicht ändert. Dies ist jedoch normalerweise nicht der Fall, da die Firefly-Reaktion einer Produktinhibierung durch Oxyluciferin unterliegt, die bei der Messung einer definierten ATP-Konzentration zu einem starken zeitlichen Signalabfall und so zu einem eher blitzartigen Signal-Zeit-Verlauf führt, der höchstens einige Sekunden lang konstant verläuft.

Es wurde nun überraschenderweise gefunden, daß das erfindungsgemäß zur Inhibierung der Adenylatkinasen zugesetzte AMP unter den angegebenen Bedingungen die Eigenschaften der Firefly-Luciferase so ändert, daß die im Verlauf der Reaktion üblicherweise auftretende Produktinhibierung durch Oxyluciferin beseitigt wird. Dies führt dazu, daß bei Messung einer definierten ATP-Konzentration anstelle eines blitzartigen Signal-Zeit-Verlaufs wie bisher eine weitgehende Signalkonstanz über mehr als 15 Minuten erreicht wird.

In der beigefügten Zeichnung zeigt Fig. 1 die erfindungsgemäß erzielte Signalkonstanz. Sie wurde mit folgendem Testansatz erstellt:

Komponente

| | |
|---|---|
| D-Luciferin | 35 µMol/l |
| Luciferase | 400 Einheiten (Einheiten/Test) |
| Tris-Acetat-Puffer | pH 7,75, 100 mMol/l |
| EDTA | 2 mMol/l |
| $MG^{++}$ | 10 mMol/l |
| ATP | $10^{-8}$ Mol/l |
| RSA | 0,1% |
| AMP | 5 mMol/l |

Ein weiterer Gegenstand der Erfindung ist ein Reagenz zur Bestimmung der Creatinkinase. Dieses ist dadurch gekennzeichnet, daß es 10 bis 500 µMol Luciferin, mindestens 50 U/Test Luciferase, 5 bis 250 mMol Puffer, pH 5,8 bis 7,5, 1 bis 10 mMol AMP, 0,1 bis 10 mMol ADP, 1 bis 100 µMol Diadenosinpentaphosphat, 5 bis 50 mMol Creatinphosphat, 1 bis 100 mMol organische Sulfhydrylverbindung, 0,1 bis 5 mMol Sequestrierungsmittel, 0,05 bis 1 Gew.-% Rinderserumalbumin und 1 bis 100 mMol Magnesiumionen für je 1 Liter Testlösung enthält. Es kann in fester oder gelöster Form vorliegen.

In einer bevorzugten Ausführungsform enthält das erfindungsgemäße Reagenz 15 bis 50 µMol Luciferin, 100 bis $5 \times 10^4$ U/Test Luciferase, 60 bis 120 mMol Puffersubstanz, pH 6,3 bis 7,2, 5 bis 10 mMol AMP, 0,5 bis 2 mMol ADP, 5 bis 50 µMol Diadenosinpentaphosphat, 20 bis 40 mMol Creatinphosphat, 2 bis 50 mMol organische Sulfhydrylverbindung, 0,5 bis 3 mMol Sequestrierungsmittel, 0,05 bis 0,2 Gew.-% Rinderserumalbumin und 5 bis 20 mMol Magnesiumionen für je 1 Liter Testlösung.

Als Sequestrierungsmittel enthält das erfindungsgemäße Reagenz vorzugsweise EDTA, als Sulfhydrylverbindung N-Acetylcystein.

Beispiele für geeignete Puffersubstanzen sind Tris-Acetat, Imidazolacetat, Hepesacetat, Tris-, Imidazol-, TRA-, Hepes- als Sulfat oder Chlorid, Arsenat- oder Phosphat-Puffer. Auch Glycinpuffer im genannten Bereich ist geeignet. Bevorzugt werden Imidazolacetat und Hepesacetat verwendet.

Die folgenden Beispiele erläutern die Erfindung weiter. Konzentrationsangaben beziehen sich auf fertige Reagenzlösung im Test, Mengenangaben auf die zur Herstellung von 1 Liter Testlösung benötigte Menge. In den Beispielen wurde eine nach dem Verfahren von FEBS-Lett. 70, 167—170 (1976) isolierte Firefly-Luciferase verwendet. Als Luciferin wurden kommerziell erhältliche Produkte des jeweils höchsten erhältlichen Reinheitsgrads, beispielsweise die Chargen Sigma 58C-0349 und 98C-3942 oder Calbiochem Charge 540022 (Handelsbezeichnungen) verwendet. In gleicher Weise eignen sich auch andere Luciferin-Präparate vergleichbarer Reinheit.

Die im folgenden angegebenen Luciferase-Einheiten wurden wie folgt ermittelt:

| Testansatz | Endkonzentration im Test |
|---|---|
| D-Luciferin | 35 µMol/l |
| Tris-Acetat, pH 7,75 | 100 mMol/l |
| EDTA | 2 mMol/l |
| RSA | 0,1% |
| Mg | 10 mMol/l |
| Luciferase | variabel |
| ATP (als Start) | $2{,}5 \times 10^{-7}$ Mol/l |

Die Messung wurde an einem handelsüblichen ATP-Photometer (Firma SAI, San Diego, Calif., USA) durchgeführt.

Als Enzymeinheit gilt diejenige Menge, die unter den oben angegebenen Bedingungen ein Signal von 37 Impulsen/10 Sekunden gibt. Hierbei war die Potentiometer-Empfindlichkeit am obigen Gerät auf 6,7 eingestellt.

Beispiel 1

| Lösungen | Konz. in Lösung | Konz. im Test |
|---|---|---|
| Lösung 1 | | |
| EDTA | 4 mMol/l | 2 mMol/l |
| Mg-Acetat | 20 mMol/l | 10 mMol/l |
| D-Luciferin | 70 µMol/l | 35 µMol/l |
| Luciferase | 400 Einheiten/ml | 400 Einheiten/Test |
| RSA | 4 g/l | 0,2% |
| Imidazolacetat, pH 6,7 | 110 mMol/l | 100 mMol/l |
| Lösung 2 | | |
| AMP | 33 mMol/l | 10 mMol/l |
| N-Acetylcystein (NAC) | 33 mMol/l | 10 mMol/l |
| Ap5A | 33 µMol/l | 10 µMol/l |
| ADP | 3,3 mMol/l | 1 mMol/l |
| Imidazolacetat, pH 6,7 | 110 mMol/l | 100 mMol/l |
| Lösung 3 | | |
| Creatinphosphat | 300 mMol/l | 30 mMol/l |
| Imidazolacetat | 110 mMol/l | 100 mMol/l |
| Lösung 4 | | |
| ATP | $5 \times 10^{-4}$ Mol/l | $5 \times 10^{-6}$ Mol/l |

Test

Es werden zusammengegeben:

1000 µl Lösung 1
600 µl Lösung 2
200 µl Serumprobe

Das Gemisch wird bei 25°C 5 Minuten vorinkubiert, dann wird das Testglas in das Meßgerät (SAI-Photometer) gestellt und die Reaktion durch Zugabe von 200 µl Lösung 3, die auch auf 25°C vorinkubiert war, gestartet. Der Kurvenverlauf wird auf einen Schreiber gegeben und nach ca. 2 Minuten wird mit 20 µl Lösung 4 die interne Eichung in µMol Substratumsatz/Minute vorgenommen. Die Ergebnisse werden von einem Schreiber automatisch aufgezeichnet. Der Kurvenverlauf wird weitere 2 bis 3 Minuten aufgezeichnet, um ein graphisch gut auswertbares Bild zu erhalten, welches in Fig. 2 der beigefügten Zeichnung dargestellt ist.

Die linear ansteigende Kurve gibt die Steigung in willkürlichen Skalenteilchen/Minute an und die Eichstufe ermöglicht die Umrechnung von Skalenteilen (als $\Delta$ mV eingetragen) in µMol ATP. Die bei der Umrechnung erhaltene Kurve ist in Fig. 3 der Zeichnung dargestellt.

Beispiel 2

Testkit für 100 Tests

Flasche 1 enthält
- 0,4 mMol EDTA
- 2 mMol Magnesiumacetat
- 7 µMol Luciferin
- $4 \times 10^4$ Einheiten Luciferase
- 400 mg Rinderserumalbumin
- 11 mMol Imidazol-Puffer, pH 6,7

als Lyophilisat.

Flasche 2 enthält
- 2 mMol Adenosin-5'-monophosphat
- 2 mMol N-Acetylcystein
- 2 µMol Diadenosinpentaphosphat
- 0,2 mMol Adenosin-5'-diphosphat
- 6,5 mMol Imidazolacetat-Puffer, pH 6,7

als Lyophilisat.

Flasche 3 enthält
- 6 mMol Creatinphosphat
- 2,2 mMol Imidazolacetat-Puffer, pH 6,7

als Lyophilisat.

Flasche 4 enthält
- $10^{-6}$ Mol ATP

als Lyophilisat.

Der Inhalt von Flasche 1 wird in 100 ml Wasser, der Inhalt von Flasche 2 in 60 ml Wasser, der Inhalt von Flasche 3 in 20 ml Wasser und der Inhalt von Flasche 4 in 2 ml Wasser aufgenommen zur Herstellung von gebrauchsfertigen Lösungen. Die Testdurchführung selbst erfogt analog Beispiel 1 unter Verwendung der in Beispiel 1 angegebenen Mengen der einzelnen Lösungen.

**Patentansprüche**

1. Verfahren zur Bestimmung von Creatinkinase durch Reaktion von Creatinphosphat mit Adenosindiphosphat unter Bildung von Adenosintriphosphat, Umsetzung des letzteren mit Luciferin und Sauerstoff in Gegenwart von Luciferase und Diadenosinpentaphosphat unter Bildung von Oxyluciferin und Adenosinmonophosphat, und Messung des dabei emittierten Lichts, dadurch gekennzeichnet, daß man die Umsetzung bei Adenosindiphosphat-Substrat-Sättigungskonzentration in Gegenwart von 1 bis 10 mMol/l AMP bei pH-Werten von 5,8 bis 7,5 mit mindestens 50 U/Test Luciferase durchführt.

2. Verfahren nach Anspruch 1, dadurch gekennzeichnet, daß man 0,1 bis 10 mMol/l ADP und 1 bis 100 µMol/l Diadenosinpentaphosphat zusetzt.

3. Verfahren nach Anspruch 2, dadurch gekennzeichnet, daß man bei pH 6,3 bis 7,2 in Gegenwart von 0,5 bis 2 mMol/l ADP, 5 bis 50 µMol/l Diadenosinpentaphosphat und 5 bis 10 mMol/l AMP arbeitet.

4. Verfahren nach einem der Ansprüche 1 bis 3, dadurch gekennzeichnet, daß man die Bestimmung in Gegenwart eines Sequestrierungsmittels, wie Äthylendiamin-tetraessigsäure, einer organischen Sulfhydrylverbindung, wie N-Acetylcystein, und von Serumalbumin durchführt.

5. Reagenz zur Bestimmung der Creatinkinase, dadurch gekennzeichnet, daß es

10 bis 500 µMol Luciferin,
mindestens 50 U/Test Luciferase,
5 bis 250 mMol Puffer, pH 5,8 bis 7,5,
1 bis 10 mMol AMP,
0,1 bis 10 mMol ADP,
1 bis 100 µMol Diadenosinpentaphosphat,
5 bis 50 mMol Creatinphosphat,
1 bis 100 mMol organische Sulfhydrylverbindung,

0,1 bis 5 mMol Sequestrierungsmittel,
0,05 bis 1 Gew.-% Rinderserumalbumin und
1 bis 100 mMol Magnesiumionen

für je 1 Liter Testlösung enthält.

6. Reagenz nach Anspruch 5, dadurch gekennzeichnet, daß es

15 bis 50 µMol Luciferin,
100 bis $5 \times 10^4$ U/Test Luciferase,
60 bis 120 mMol Puffersubstanz, pH 6,3 bis 7,2,
5 bis 10 mMol AMP,
0,5 bis 2 mMol ADP,
5 bis 50 µMol Diadenosinpentaphosphat,
20 bis 40 mMol Creatinphosphat,
2 bis 50 mMol organische Sulfhydrylverbindung,
0,5 bis 3 mMol Sequestrierungsmittel,
0,05 bis 0,4 Gew.-% Rinderserumalbumin und
5 bis 20 mMol Magnesiumionen

für je 1 Liter Testlösung enthält.

7. Reagenz nach Anspruch 5 oder 6, dadurch gekennzeichnet, daß es als Sequestrierungsmittel Äthylendiamin-tetraessigsäure enthält.

8. Reagenz nach einem der Ansprüche 5 bis 7, dadurch gekennzeichnet, daß es als Sulfhydrylverbindung N-Acetylcystein enthält.

9. Reagenz nach einem der Ansprüche 5 bis 8, dadurch gekennzeichnet, daß es als Puffersubstanz Imidazolacetat oder Hepesacetat enthält.

## Claims

1. Process for the determination of creatine kinase by the reaction of creatine phosphate with adenosine diphosphate with the formation of adenosine triphosphate, reaction of the latter with luciferin and oxygen in the presence of luciferase and diadenosine pentaphosphate with the formation of oxyluciferin and adenosine monophosphate and measurement of the light thereby emitted, characterised in that one carries out the reaction at the adenosine diphosphate substrate saturation concentration in the presence of 1 to 10 mMol/litre AMP at pH values of 5.8 to 7.5 with at least 50 U/test of luciferase.

2. Process according to claim 1, characterised in that one adds 0.1 to 10 mMol/litre ADP and 1 to 100 µMol/litre diadenosine pentaphosphate.

3. Process according to claim 2, characterised in that one operates at pH 6.3 to 7.2 in the presence of 0.5 to 2 mMol/litre ADP, 5 to 50 µMol/litres diadenosine pentaphosphate and 5 to 10 mMol/litre AMP.

4. Process according to one of claims 1 to 3, characterised in that one carries out the determination in the presence of a sequestering agent, such as ethylenediamine-tetraacetic acid, of an inorganic sulphhydryl compound, such as N-acetylcysteine, and of serum albumin.

5. Reagent for the determination of creatine kinase, characterised in that it contains

10 to 500 µMol luciferin,
at least 50 U/test luciferase,
5 to 250 mMol buffer, pH 5.8 to 7.5,
1 to 10 mMol AMP,
0.1 to 10 mMol ADP,
1 to 100 µMol diadenosine pentaphosphate,
5 to 50 mMol creatine phosphate,
1 to 100 mMol organic sulphhydryl compound,
0.1 to 5 mMol sequestering agent,
0.05 to 1 wt.% bovine serum albumin and
1 to 100 mMol magnesium ions

for each 1 litre of test solution.

6. Reagent according to claim 5, characterised in that it contains

15 to 50 µMol luciferin,
100 to $5 \times 10^4$ U/test luciferase,
60 to 120 mMol buffer substance, pH 6.3 to 7.2,
5 to 10 mMol AMP,

0.5 to 2 mMol ADP,
5 to 50 μMol diadenosine pentaphosphate,
20 to 40 mMol creatine phosphate,
2 to 50 mMol organic sulphhydryl compound,
0.5 to 3 mMol sequestering agent,
0.05 to 0.4 wt.% bovine serum albumin and
5 to 20 mMol magnesium ions

for each 1 litre of test solution.

7. Reagent according to claim 5 or 6, characterised in that it contains ethylenediamine-tetraacetic acid as sequestering agent.

8. Reagent according to one of claims 5 to 7, characterised in that it contains N-acetylcysteine as sulphhydryl compound.

9. Reagent according to one of claims 5 to 8, characterised in that it contains imidazole acetate or hepes acetate as buffer substance.

**Revendications**

1. Procédé pour le dosage de la créatinekinase par réaction du phosphate de créatine avec le diphosphate d'adénosine avec formation de triphosphate d'adénosine, réaction de ce dernier avec la luciférine et l'oxygène en présence de luciférase et de pentaphosphate de diadénosine avec formation d'oxyluciférine et de monophosphate d'adénosine, et mesure de la lumière alors émise, caractérisé en ce qu'on effectue la réaction à la concentration de saturation du diphosphate d'adénosine en tant que substrat, en présence de 1 à 10 mmoles/l d'AMP à des valeurs de pH de 5,8 à 7,5 avec au moins 50 U/essai de luciférase.

2. Procédé selon la revendication 1, caractérisé en ce qu'on ajoute 0,1 à 10 mmoles/l d'ADP et 1 à 100 μmoles/l de pentaphosphate de diadénosine.

3. Procédé selon la revendication 2, caractérisé en ce qu'on travaille à pH 6,3 à 7,2 en présence de 0,5 à 2 mmoles/l d'ADP, 5 à 50 μmoles/l de pentaphosphate de diadénosine et 5 à 10 mmoles/l d'AMP.

4. Procédé selon l'une des revendications 1 à 3, caractérisé en ce qu'on effectue le dosage en présence d'un agent séquestrant tel que l'acide éthylènediaminetétracétique, d'un composé organique sulfhydrylé tel que la N-acétylcystéine, et d'albumine du sérum.

5. Réactif pour le dosage de la créatinekinase, caractérisé en ce qu'il contient

10 à 500 μmoles de luciférine,
au moins 50 U/essai de luciférase,
5 à 250 mmoles de tampon, pH 5,8 à 7,5,
1 à 10 mmoles d'AMP,
0,1 à 10 mmoles d'ADP,
1 à 100 μmoles de pentaphosphate de diadénosine,
5 à 50 mmoles de phosphate de créatine,
1 à 100 mmoles de composé organique sulfhydrylé,
0,1 à 5 mmoles d'agent séguestrant,
0,05 à 1% en poids d'albumine du sérum de bovin et
1 à 100 mmoles d'ions magnésium

par litre de solution d'essai.

6. Réactif selon la revendication 5, caractérisé en ce qu'il contient

15 à 50 μmoles de luciférine,
100 à $5 \times 10^4$ U/essai de luciférase,
60 à 120 mmoles de substance tampon, pH 6,3 à 7,2,
5 à 10 mmoles d'AMP,
0,5 à 2 mmoles d'ADP,
5 à 50 μmoles de pentaphosphate de diadénosine,
20 à 40 mmoles de phosphate de créatine,
2 à 50 mmoles de composé organique sulfhydrylé,
0,5 à 3 mmoles d'agent séquestrant,
0,05 à 0,4 % en poids d'albumine du sérum de bovin et
5 à 20 mmoles d'ions magnésium

par litre de solution d'essai.

7. Réactif selon la revendication 5 ou 6, caractérisé en ce qu'il contient, en tant qu'agent séquestrant, de l'acide éthylènediaminetétracétique.

8. Réactif selon l'une des revendications 5 à 7, caractérisé en ce qu'il contient, en tant que composé sulfhydrylé, de la N-acétylcystéine.

9. Réactif selon l'une des revendications 5 à 8, caractérisé en ce qu'il contient, en tant que substance tampon, de l'acétate d'imidazole ou de l'acétate d'hepes.

FIG. 1

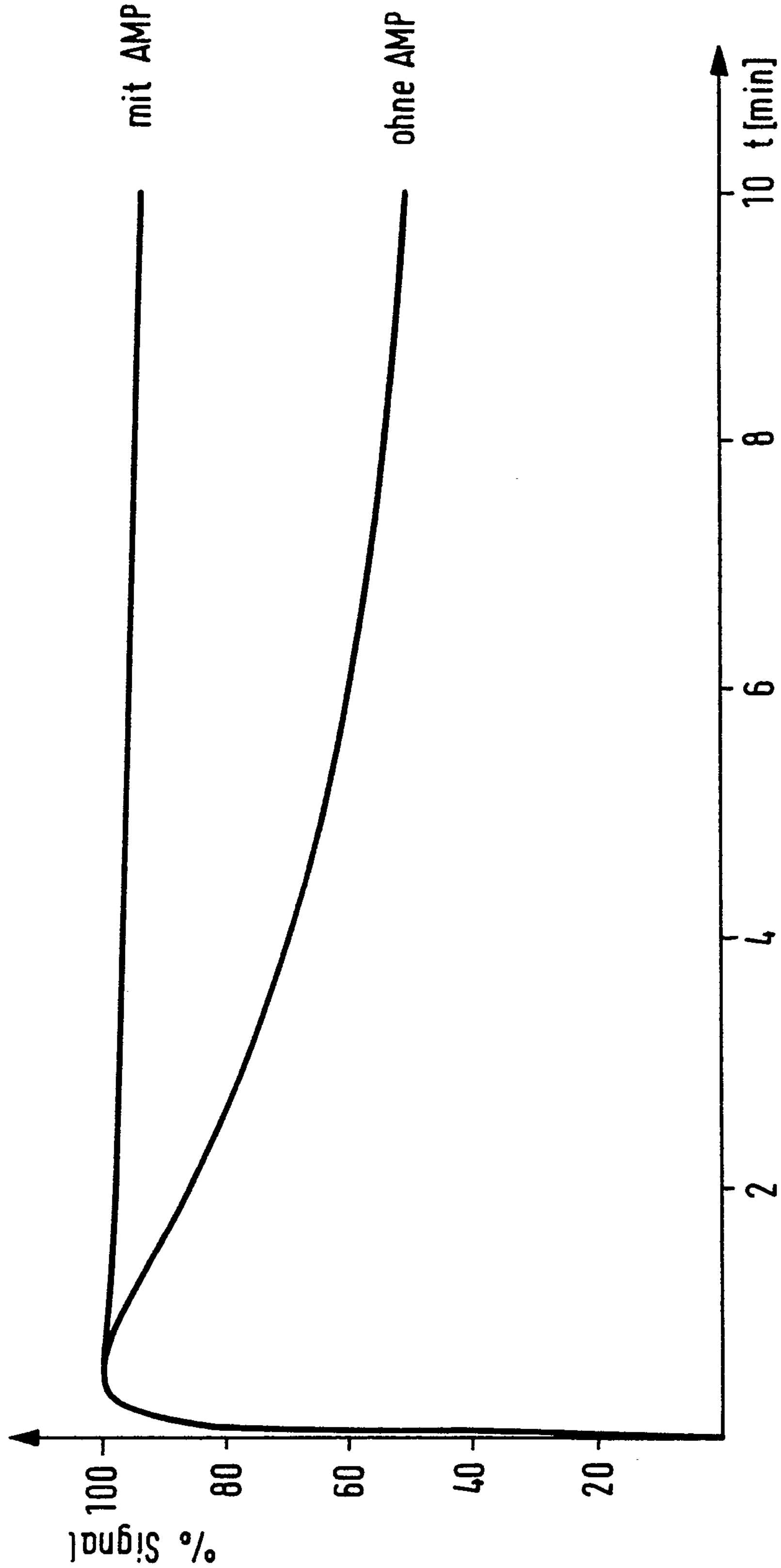

FIG. 2

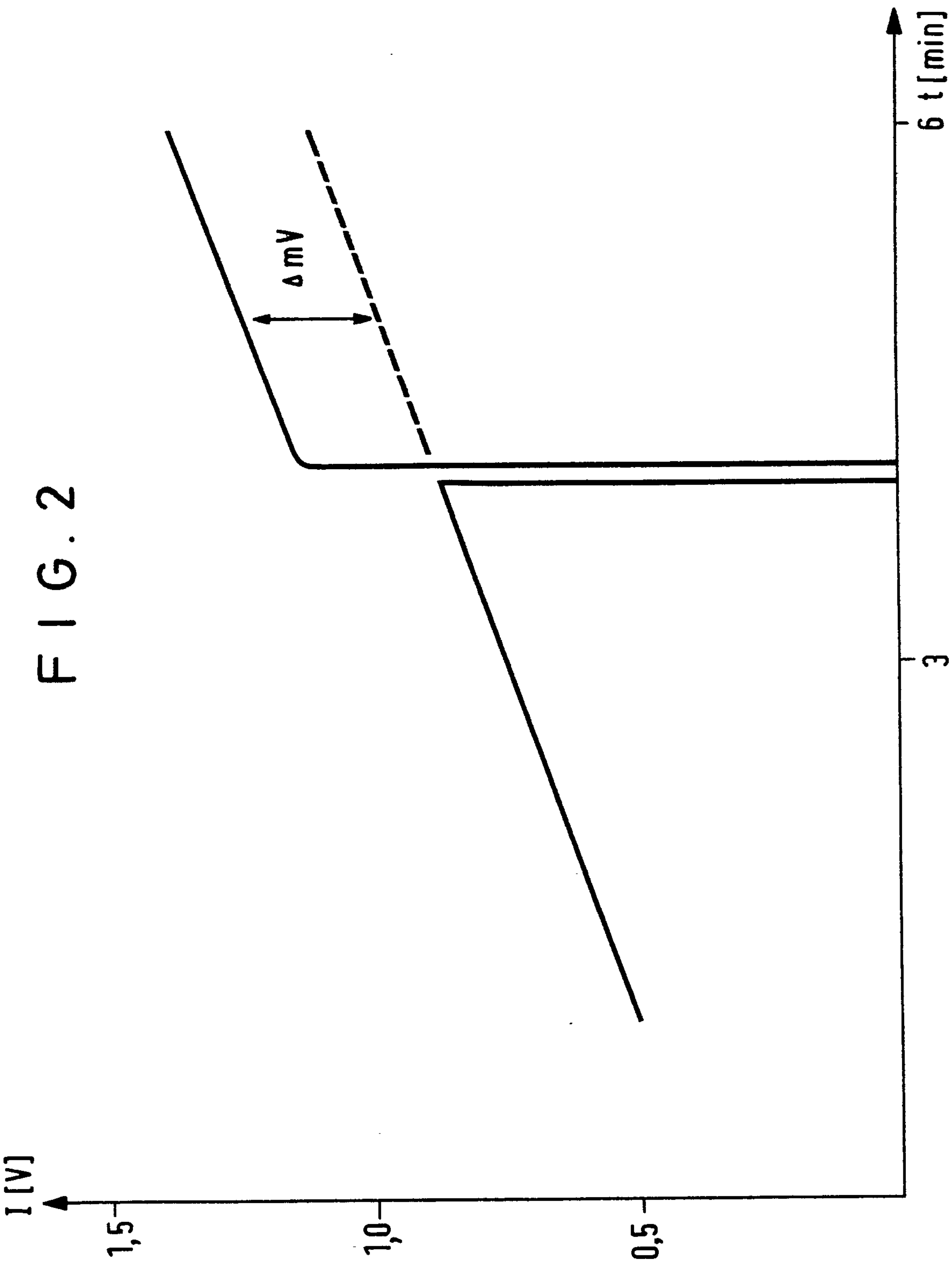

FIG. 3

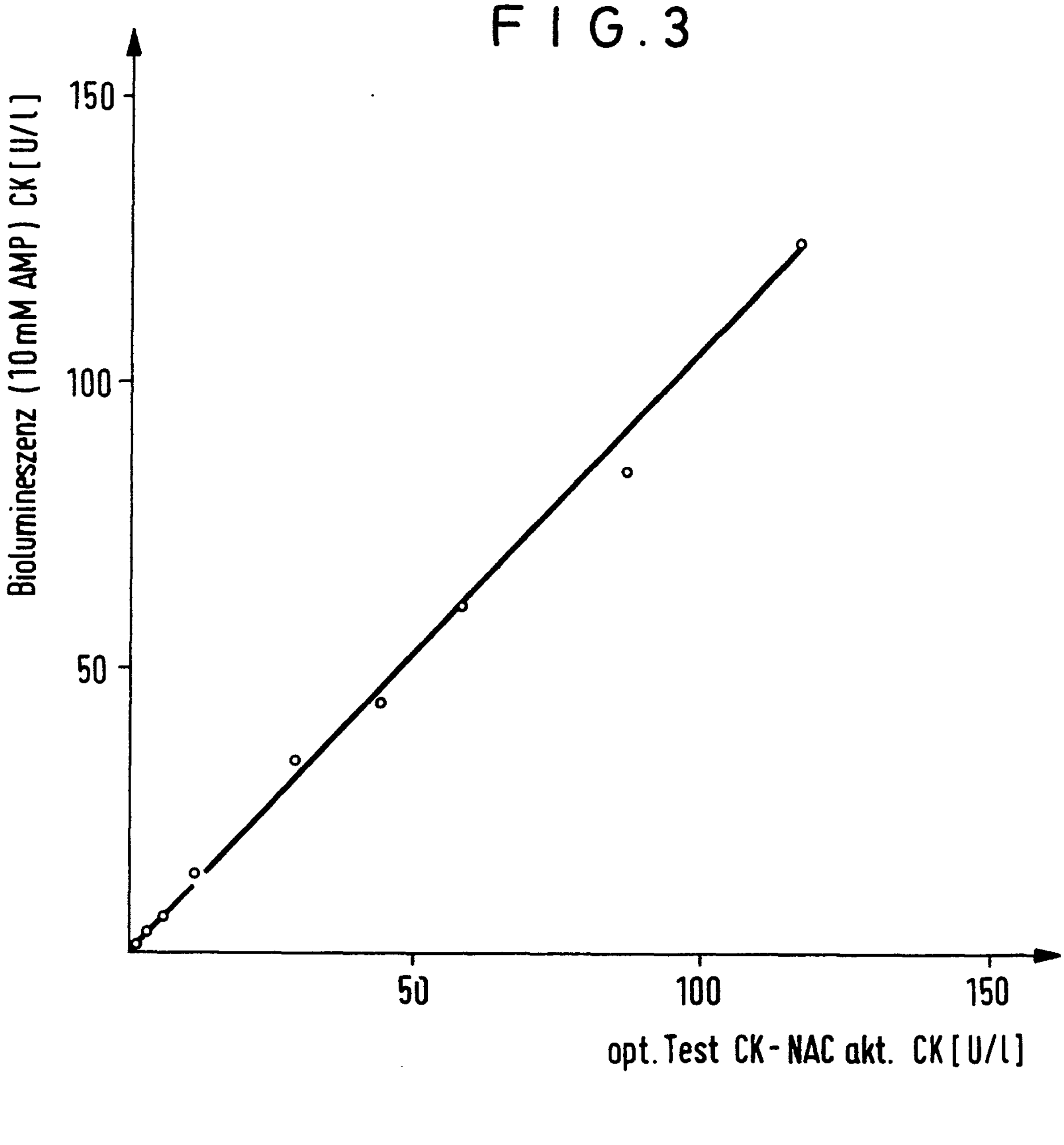
150
100
50
Biolumineszenz (10 mM AMP) CK [U/l]
50
100
150
opt. Test CK-NAC akt. CK [U/l]